# EUROPEAN PATENT SPECIFICATION

(11) **EP 0 725 776 B1**
(45) Date of publication and mention of the grant of the patent: **16.07.1997**
(21) Application number: 94930582.5
(22) Date of filing: 04.10.1994
(51) Int. Cl.: C07D 249/08, A61K 31/41

(54) **3-ARYL-4-ALKYL AND 4,5-DIALKYL-4H-1,2,4-TRIAZOLES USEFUL AS MEMORY ENHANCERS**
3-ARYL-4-ALKYL UND 4,5-DIALKYL-4H-1,2,4-TRIAZOLE, VERWENDBAR GEGEN GEDÄCHTNIS-STÖRUNGEN
3-ARYL-4-ALKYL ET 4,5-DIALKYL-4H-1,2,4-TRIAZOLES PERMETTANT D'AMELIORER LA MEMOIRE

(30) Priority: 29.10.1993 US 146373
(43) Date of publication of application: 14.08.1996
(73) Proprietor: MERRELL PHARMACEUTICALS INC., Cincinnati, Ohio 45215-6300 (US)
(72) Inventor: DALTON, Christopher, R., Cincinnati, OH 45236 (US); KANE, John, M., Cincinnati, OH 45236 (US); MILLER, Jerry, A., Fairport, NY 14450 (US)
(74) Representative: VOSSIUS & PARTNER
(86) International application number: US9411255
(87) International publication number: WO9511887

(56) References cited:
- EP-A- 0 221 485
- EP-A- 0 452 926
- DE-C- 541 700
- JOURNAL OF MEDICINAL CHEMISTRY, vol.14, no.3, March 1971, WASHINGTON US pages 260 - 262 M.Y. MHASALKAR ET AL. 'Further studies in substituted 4H-1,2,4-triazoles for possible hypoglycemic activity'

## Description

This invention relates to novel 3-aryl-4-alkyl and 4,5-dialkyl-4H-1,2,4-triazoles and to 3-aryl-4-alkyl and 4,5-dialkyl-4H-1,2,4-triazoles for use as enhancers of cognition and memory.

More specifically, this invention relates to compounds of the formula I and the pharmaceutically acceptable salts thereof for administration in the enhancement of memory and cognition and the treatment of age-related memory deficit, Alzheimer's disease and Wernicke-Korsakoff syndrome, 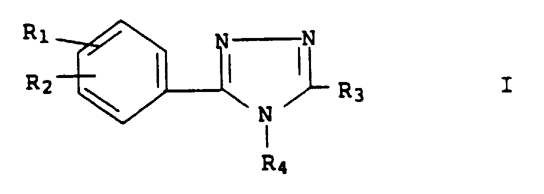 wherein
R₁ and R₂ independently represent hydrogen, halogen, trifluoromethyl, nitro, C₁₋₄ lower alkyl or C₁₋₄ lower alkoxy,
or, together, R₁ and R₂ represent -CH=CH-CH=CH-, forming a 1- or 2-naphthylenyl ring system;
R₃ represents hydrogen or C₁₋₄ lower alkyl; and
R₄ represents C₁₋₄ lower alkyl, benzyl, or benzyl substituted by one or two groups selected from halogen, trifluoromethyl, nitro, C₁₋₄ lower alkyl or C₁₋₄ lower alkoxy.

In addition, the invention relates to novel 3-aryl-4-alkyl and 4,5-dialkyl-4H-1,2,4-triazoles of the formula 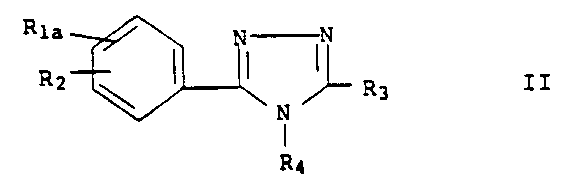 wherein
R₁ₐ represents halogen, trifluoromethyl, nitro, C₁₋₄ lower alkyl or C₁₋₄ lower alkoxy; and R₂ represents hydrogen, halogen, trifluoromethyl, nitro, C₁₋₄ lower alkyl or C₁₋₄ lower alkoxy,
or, together, R₁ₐ and R₂ represent -CH=CH-CH=CH-,
forming a 1- or 2-naphthylenyl ring system; R₃ represents hydrogen or C₁₋₄ lower alkyl; and
R₄ represents C₁₋₄ lower alkyl, benzyl, or benzyl substituted by one or two groups selected from halogen, trifluoromethyl, nitro, C₁₋₄ lower alkyl or C₁₋₄ lower alkoxy,
with the proviso that when R₁ₐ represents 4-chloro and R₂ and R₃ both represent hydrogen, R₄ is other than ethyl.

### BACKGROUND OF THE INVENTION

Memory is dependent upon the function of cholinergic cells in the cortex and hippocampus of the forebrain. The cholinergic cells in the basal forebrain reside in three regions, the nucleus basalis of Meynert, the medial septal nucleus and the nucleus of the diagonal band. These cells are responsible for most, perhaps all, of the cholinergic innervation in the cortex and hippocampus. It is known that these three structures and their respective pathways are important in memory. Additionally, it is known that up to half of these neurons and their projections may be lost in Alzheimer's dementia. By stimulating the remaining neurons it is possible to recover some of the memory deficits in Alzheimer's dementia and other forms of memory loss, including Wernicke-Korsakoff syndrome.

Previous reports have indicated that agents with activity at the γ-aminobutyric acid (GABA)-receptor complex when given in vivo modulate high affinity choline uptake (HACU) measured in vitro. It is thought that HACU measured in vitro reflects the activity of cholinergic neurons in vivo. Drugs which have a sedative or hypnotic activity have generally been found to depress cortical or hippocampal HACU. More recently, several studies, for example, those of Lorez, et al., Drug Devel. Res. 14, 359-362, 1988; Shih and Pugsley, Life Sci. 36, 2145-2152, 1985; Spignoli et al., Clin. Neuropharmacol. Supp. 3, 39-47, 1986; Nakahiro, M., et al., Br. J. Pharmacol. 95, 1303-1307, 1988, report that drugs which enhance cognition, e.g., pramiracetam, oxiracetam and pantoyl-GABA, stimulate cortical or hippocampal HACU after in vivo administration.

Another measure of cholinergic activity is the binding of the radioligand [³H] hemicholinium-3, ([³H] HC-3) which labels the carrier that mediates choline transport. Swann and Hewitt (Neuropharmacol. 27:611-615, 1988) have demonstrated that the Bₘₐₓ of [³H] HC-3 increases in parallel with HACU when cholinergic synaptosomes are stimulated. Therefore, the stimulation of [³H] HC-3 binding in vitro after treatment with drugs in vivo is also a marker for increased cholinergic activity, predictive of enhanced cognition in treated animals.

Compounds having a wide variety of chemical structures have been reported in the prior art to have cognition enhancing activity and to be useful for treatment of Alzheimer's disease. Unfortunately, most of the known memory enhancing compounds also produce side effects which limit their therapeutic potential. Such side effects have not been found with compounds of formula I. Among compounds known to have cognition enhancing activity are 5-aryl-4-alkyl-3H-1,2,4-triazole-3-thiones, which differ from compounds of Formula I in that they carry a thione moiety on a triazole ring carbon atom and an additional N-alkyl substituent. The use of these triazole-3-thiones for treatment of Wernicke-Korsakoff syndrome and Alzheimer's disease and for enhancement of cognition is described in U. S. patent 5,100,906, issued March 31, 1992, and U. S. patent 5,236,942, issued August 17, 1993. Unlike the compounds of formulae I and II, however, these triazole-3-thiones have additional activity as antidepressants, as disclosed, for example, in U. S. patent 4,775,688, issued October 4, 1988, and in U. S. patent 4,912,095, issued March 27, 1990.

3-(4-Chlorophenyl)-4-methyl-4H-1,2,4-triazole, which was reported to have hypoglycemic activity by M. Y. Mhasalkar, et al., J. Med. Chem 14(3) 260-262, (1971), is the only compound of formula I to have been administered to an animal as a medicament.

### Detailed Description of the Invention

In compounds of formulae I and II wherein one of R1 or R₁ₐ and R₂ is hydrogen, the mono-substituted phenyl moiety carries the R-substitutent at any of the ortho, meta or para positions; when each of R₁ or R₁ₐ and R₂ is halogen, trifluoromethyl, nitro, C₁₋₄ lower alkyl or C₁₋₄ lower alkoxy, the disubstituted phenyl moiety is substituted in any of the 2,3-; 2,4-; 2,5-; 2,6-; 3,4-; and 3,5-positions. As used herein halogen represents chloro, fluoro, bromo or iodo. In preferred compounds of formula I, R₁ is other than hydrogen and R₂ is hydrogen, i.e., the preferred compounds include a monosubstituted phenyl moiety. Preferably R₁ represents halogen, with fluoro being most preferred. When R₁ or R₂ represents C₁₋₄ alkyl or C₁₋₄ alkoxy, the alkyl moiety may be straight or branched. Compounds wherein R₃ is hydrogen are preferred, and R₄ preferably represents methyl. R₃ and R₄ may independently represent any straight or branched C₁₋₄ alkyl group.

The pharmacological properties of these compounds as enhancers of memory and cognition and their relative potencies may be measured through their effect on neuro-transmitters in the brain. Since drugs that block GABA inhibition in the cholinergic neurons of the basal forebrain nuclei will stimulate cholinergic firing, thus stimulating memory, the capacity of the drugs to enhance cognition can be assessed by measuring the increase in cholinergic firing rate. The increase in cholinergic firing rate is measured indirectly by measuring choline uptake or [³H] hemicholinium-3 binding in brain cells taken from treated animals.

To test for [³H] hemicholinium-3 binding in brain cells from the brain cortex, drugs were dissolved in saline by sonication. Male Sprague-Dawley rats were dosed i.p. and sacrificed by decapitation 60 min after injection. The brains were removed and dissected, and tissue was homogenized in 20 volumes of ice-cold buffer and stored frozen until assayed. Binding was measured by incubating the tissue with varying concentrations of [³H]hemicholinium-3 in an isotonic Tris buffer (pH 7.4) for 60 min at room temperature. The incubation was terminated by rapid filtration through Whatman GF/B filters. After drying, the filters were placed in scintillation cocktail and radioactivity was determined using a Beckman scintillation counter. The values for the K_{d} and Bₘₐₓ were determined by nonlinear curve-fitting and the average values for samples of 3 or more animals reported. As shown in the following table, 3-(3-fluorophenyl)-4-methyl-4H-1,2,4-triazole, a compound of formula I, increased [³H] hemicholinium-3 binding in brain cortex cells by 45% over the binding seen when saline was administered as a control. This increase in Bₘₐₓ is indicative of greatly enhanced cognition.

**TABLE 1**

| EFFECT OF IN VIVO ADMINISTRATION ON [³H] HEMICHOLINIUM-3 BINDING IN VITRO IN RAT CORTICAL MEMBRANES | | |
|---|---|---|
| Treatment | Bₘₐₓ ± SEM (fmol/mg Protein) | % Increase in Bₘₐₓ |
| Saline (n=10) | 14.10±1.81 | |
| 3-(3-Fluorophenyl)-4-methyl-4H-1,2,4-triazole (1/mg/kg),(n=10) | 20.4±2.48 | 45 |

The activity of compounds of formula I in enhancing spacial learning ability and cognition can be tested by studying their ability to reverse a water maze learning impairment induced by the benzodiazepine, diazepam (R. G. M. Morris, Learning and Motivation 12, 239-260 (1982); M. P. Arolfo, and J. D. Brioni, Behavioral and Neural Biology 55, 131-6 (1991); R. K. McNamara and R. W. Skelton, Pharmacology, Biochemistry & Behavior 38, 651-8 (1991); R. K. McNamara and R. W. Skelton, Psychopharmacology 107, 347-51 (1992)). Diazepam has been shown to produce learning and memory impairments in humans as well as in animals (R. G. Lister, Neuroscience and Biobehavioral Reviews 9, 87-94 (1985); M. Theibot, Neuroscience and Biobehavioral Reviews 9, 95-100 (1985)).

Male Sprague-Dawley rats are trained in a 120-cm diameter water-filled tank to locate a hidden platform submerged just below the surface of the water. The location of the platform remained constant, but for each trial the animal was required to swim from one of three different starting locations around the edge of the tank. There were no proximal cues in the tank, so the animal had to use a spatial mapping strategy using the distal cues around the room to navigate to the hidden platform. The animals were given 9 successive training trials during the single training day. Each trial had a maximum duration of 60 seconds. If the animal did not locate the platform by that time, it was placed on the platform. After the animal found or was placed on the platform, it was allowed to stay there for 30 seconds. The next trial commenced immediately following the 30 second stay on the platform. Latency to locate the platform was recorded for each trial using a computerized video tracking system for automated acquisition of the data.

Separate treatment groups of four animals each were run in each experiment. The results of two experiments were combined so that 8 rats were tested in each treatment group. The vehicle-vehicle group received vehicle (distilled water plus Tween) i.p. 60 minutes prior to the first trial and vehicle i.p. 20 minutes prior to the first trial. The vehicle-diazepam group received vehicle i.p. 60 minutes prior to the first trial and 2.5 mg/kg diazepam i.p. 20 minutes prior to the first trial. Two groups of animals were treated with 3-(3-fluorophenyl)-4-methyl-4H-1,2,4-triazole, a compound of formula I, prior to treatment with diazepam. One group received 20 mg/kg of 3-(3-fluorophenyl)-4-methyl-4H-1,2,4-triazole i.p. 60 minutes prior to the first trial and 2.5 mg/kg diazepam i.p. 20 minutes prior to the first trial, while the second group received 40 mg/kg of 3-(3-fluorophenyl)-4-methyl-4H-1,2,4-triazole i.p. 60 minutes prior to the first trial and 2.5 mg/kg diazepam i.p. 20 minutes prior to the first trial.

The latency scores for each animal were averaged into three blocks of three trials each (one trial from each starting location). A one-way ANOVA comparing the treatment groups was computed on the scores for each trial block. If the overall ANOVA was statistically significant, comparisons between individual treatment groups were made with Fisher's PLSD test.

**Table 2**

| Effect on Diazepam-Induced Water Maze Learning Impairment | | | |
|---|---|---|---|
| | Mean Latency (seconds) ± S.E.M. | | |
| Treatment | Block 1 | Block 2 | Block 3 |
| Vehicle,vehicle | 41.92±1.10 | 20.61±3.94 | 21.23±5.32 |
| Vehicle,diazepam 2.5 mg/kg | 57.70±2.30 | 49.67±6.06 | 55.76±4.24 |
| 3-(3-Fluorophenyl)-4-methyl-4H-1,2,4-triazole, 20 mg/kg, diazepam 2.5 mg/kg | 44.68±2.56 | 37.67±6.01 | 35.21±6.00 |
| 3-(3-Fluorophenyl)-4-methyl-4H-1,2,4-triazole, 40 mg/kg, diazepam 2.5 mg/kg | 58.19±1.01 | 52.83±3.85 | 53.59±4.31 |

The data in Table 2 show that 3-(3-fluorophenyl)-4-methyl-4H-1,2,4-triazole attenuated diazepam-induced impairment at 20 mg/kg, but not at 40 mg/kg, indicating that this compound has a bell-shaped dose-response curve, with activity at intermediate doses, but not at high or low doses. This is a common finding with potential cognition-enhancing compounds. The overall ANOVAs for all three trial blocks were significant, F(3, 28) = 20.649, p = .0001 for block 1, F (3, 28) = 8.3, p < .001 for block 2, and F (3, 28) = 10.577, p = .0001 for block 3. Individual comparisons indicated that the vehicle-diazepam group differed significantly from the vehicle-vehicle group (p < .05) on all three blocks, indicating that diazepam significantly impaired water maze learning. The group that received 40 mg/kg 3-(3-fluorophenyl)-4-methyl-4H-1,2,4-triazole plus diazepam also differed significantly from the vehicle-vehicle group (p < .05) on all three blocks, indicating that the 40 mg/kg dose did not affect the diazepam-induced impairment. In contrast, the group that received 20 mg/kg 3-(3-fluorophenyl)-4-methyl-4H-1,2,4-triazole plus diazepam was not significantly different from the vehicle-vehicle group except on block 2 (p < .05), and was significantly different from the vehicle diazepam group on blocks 1 and 3 (p < .05),indicating that the 20 mg/kg dose attenuated the diazepam-induced impairment. In addition, the 20 mg/kg group was also significantly different from the 40 mg/kg group on all three blocks (p < .05). These results indicate that 3-(3-fluorophenyl)-4-methyl-4H-1,2,4-triazole has cognition-enhancing effects and can be used at appropriate dosages to treat cognitive deficits.

Compounds of formula I can be administered to mammalian patients, including humans, afflicted with cognitive disorders such as Alzheimer's disease and other forms of memory loss. In addition to Alzheimer's disease, other types of dementia that display cholinergic deficits may be ameliorated by compounds of formula I. For example, Wernicke-Korsakoff syndrome, a form of dementia resulting from alcoholism, can also be treated by administration of a cognition-enhancing dosage of compound of formula I. Arendt, et al., Acta Neuropathologica 61:101-108, 1983, have found indications that some patients with Wernicke-Korsakoff syndrome have significant loss of cholinergic neurons in the basal forebrain in addition to adrenergic deficits.

Normal aging may result in a generalized deficit in cholinergic function even in the absence of dementia. Sherman, et al., Neurobiol Aging 2:99-104, 1981, found choline uptake in aged (23-26 month old) rats to be decreased by 22% when compared to young adult rats (6 months old). This decrease in cholinergic activity was observed without any concomitant loss of cholinergic neuron number. Animal research suggests that enhancement of memory may be possible in non-demented individuals as well. Micheau, et al., Pharmacol. Biochem. Behav. 23:195-198, 1985, found that in mice trained in an operant conditioning memory task, performance was enhanced in mice treated with sulbutiamine, which increased hippocampal high affinity choline uptake, versus normal vehicle-treated control mice. Indeed, mice trained in several different memory paradigms exhibit an increase in high affinity choline uptake in cortex and hippocampus, as shown by Toumane, et al., Behav. Brain Res. 30:225-234, 1988, suggesting that such an increase in cholinergic activity in these regions is a normal part of memory formation. Treatment of normal aged individuals with a compound of formula I will enhance memory by counteracting the cholinergic deficit that interferes with learning.

For oral administration, the compounds can be formulated into solid or liquid preparations such as capsules, pills, tablets, troches, powders, solutions, suspensions or emulsions. The solid unit dosage forms can be a capsule which can be of the ordinary gelatin type containing, for example, lubricants and inert filler, such as lactose, sucrose or cornstarch. In another embodiment, the compounds of general formula I can be tableted with conventional tablet bases such as lactose, sucrose and cornstarch, in combination with binders, such as acacia, cornstarch or gelatin, disintegrating agents such as potato starch or alginic acid, and a lubricant such as stearic acid or magnesium stearate.

For parenteral administration, the compounds may be administered as injectable dosages of a solution or suspension of the compound in a physiologically acceptable diluent with a pharmaceutical carrier which can be a sterile liquid such as water, alcohols, oils and other acceptable organic solvents, with or without the addition of a surfactant and other pharmaceutically acceptable adjuvants. Illustrative of oils which can be employed in these preparations are those of petroleum, animal, vegetable, or synthetic origin, for example, peanut oil, soybean oil and mineral oil. In general, water, saline, aqueous dextrose and related sugar solutions, ethanol and glycols such as propylene glycol or polyethylene glycol, or 2-pyrrolidone are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert material such as biodegradable polymers or synthetic silicones, for example Silastic®, a silicone rubber manufactured by the Dow-Corning Corporation.

As is true in many classes of compounds generally suitable for any particular pharmacological activity having a therapeutic end-use application, certain subgeneric groups and certain specific members of the class are preferred because of their overall therapeutic index and their biochemical and pharmacological profile. In this instance the preferred compounds are those wherein R₃ is hydrogen and R₄ is methyl, and those wherein the R₁ or R₁ₐ substituent is fluoro. A specifically preferred compound is 3-(3-fluorophenyl)-4-methyl-4H-1,2,4-triazole.

The compounds of formula Ia wherein R₃ is hydrogen may be prepared by desulfurizing the corresponding 5-aryl-4-alkyl-3H-1,2,4-triazole-3-thiones of formula VII, which are readily prepared using processes and procedures analogously known in the art, as seen by the following reaction scheme A. 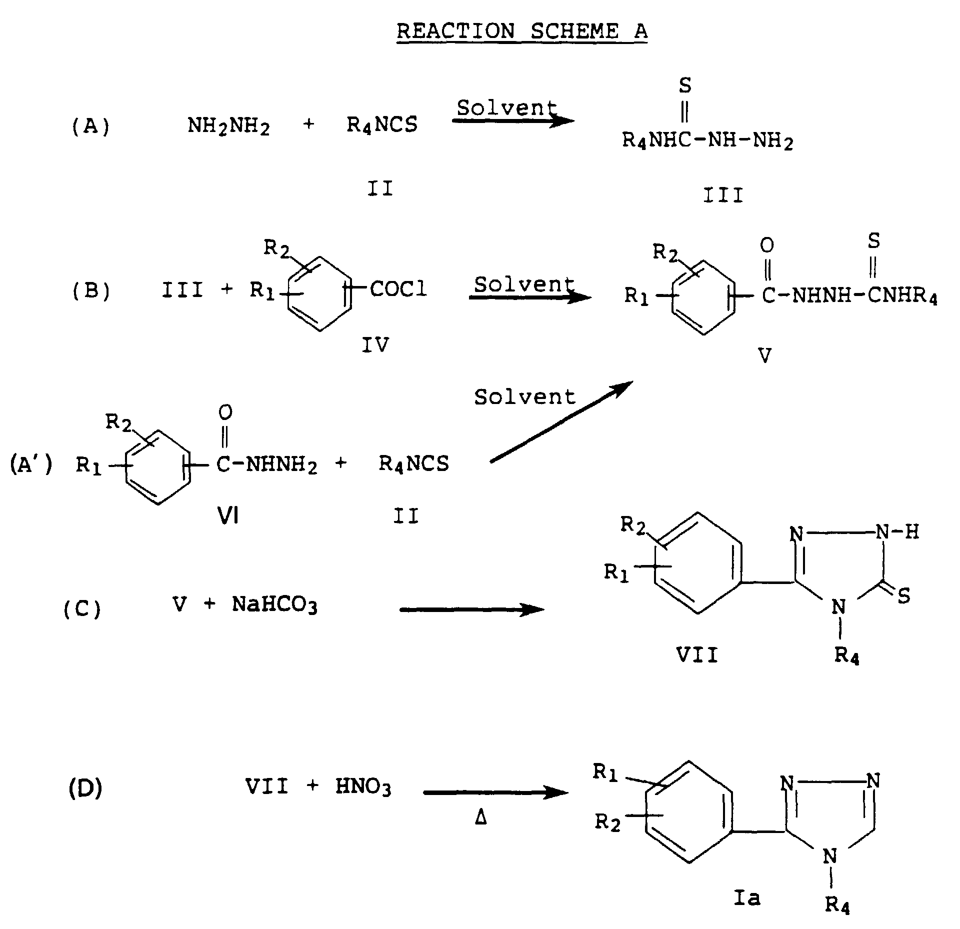 wherein R₁, R₂ and R₄ are as previously defined.

In step A, the preparation of the thiosemicarbazide (III) is readily effected by reacting hydrazine with an isothiocyanate (II) by contacting the reactants in a suitable solvent. The reaction is quite rapid and may be carried out at 0°C to room temperature. Although the reaction proceeds rapidly, the mixture may be left for up to 24 hours without significant decrease in yields. Reflux conditions may be employed but are not preferred. Almost all solvents (with the exception of water and organic acids) may be used. Anhydrous alcohols (preferably ethanol or methanol) are preferred although dimethylformamide (DMF), CHCl₃, CH₂Cl₂, tetrahydrofuran (THF) and Et₂O may also be used. Hydrazine and the required isothiocyanates are usually commercially available, but may be prepared by known techniques.

In Step B, the desired substituted aroyl thiosemicarbazides (V) may be prepared by reacting the thiosemicarbazides (III) with an R₁,R₂-substituted benzoyl chloride (IV) in an aprotic solvent such as pyridine, CHCl₃, THF or the like. The acylation proceeds rather easily at temperatures ranging from 0°C to room temperature over periods of 3 to 24 hours, although elevated temperatures (e.g. reflux temperatures) may be employed.

Alternatively, the desired substituted aroyl thiosemicarbazides (V) may be prepared in one step according to Step A', by reacting the isothiocyanate (II) with an appropriately substituted benzoic acid hydrazide of formula VI in the presence of a suitable solvent, such as THF. The reaction is effected by heating to the reflux temperature of the solvent for about 1 to 3 hours.

Again, the acid halides (IV) and the benzoic acid hydrazides (VI) are generally commercially available but may also be prepared from the corresponding acids which are generally commercially available.

In Step C, the aroyl thiosemicarbazides (V) are subjected to a cyclization reaction which is effected by heating the compounds (V) in an aqueous base, e.g. sodium bicarbonate or sodium hydroxide. Alcoholic bases may be utilized, but generally are less desirable. The reaction is conducted at about the reflux temperature of the solvent, preferably at about 65°-100°C. In practice, the thiosemicarbazides (V) need not be purified for use in Step C so that even 1:1 mixtures with pyridine hydrochloride, produced as a by-product when pyridine is employed as a solvent in Step B, may be used.

In Step D, the triazole-3-thione (VII) is desulfurized by reaction with 17% aqueous HNO₃. The reaction mixture is heated to reflux for about 30 minutes to about 1 hour, and allowed to cool to room temperature before being basified to about pH 14 with a strong aqueous base, for example KOH. The triazole of formula (Ia) is then isolated by conventional methods. For example, the aqueous reaction mixture is extracted with a suitable organic solvent, such as dichloromethane. The combined organic extracts are dried over anhydrous magnesium sulfate, filtered and concentrated under vacuum. The residue can then be recrystallized from a suitable organic solvent mixture such as acetone/hexane to provide the triazole of formula (Ia), i.e., a triazole of formula I wherein R₃ is hydrogen.

The triazoles of formula (Ib) wherein R₃ is C₁₋₄ lower alkyl can be prepared as described in Reaction Scheme B. All substituents, unless otherwise indicated, are as previously defined. The reagents and starting materials are readily available to one of ordinary skill in the art.

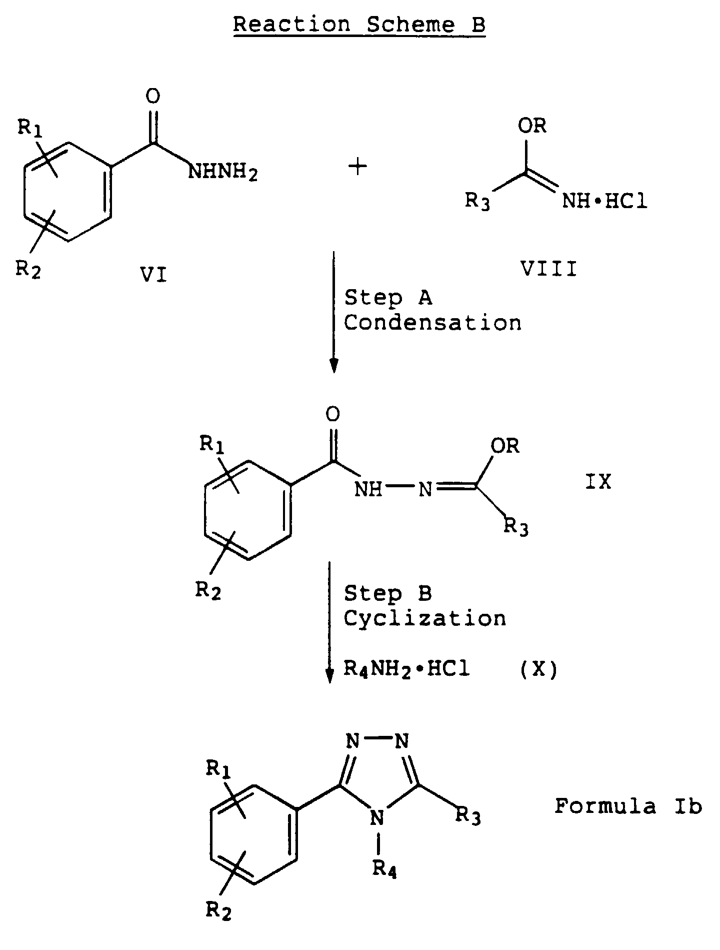

In Reaction Scheme B, step A, the benzoic acid hydrazide described by structure (VI) is subjected to a condensation reaction with the alkyl imidate hydrochloride of structure (VIII), wherein R represents a lower alkyl group, preferably methyl or ethyl, to provide the condensation product described by structure (IX). For example, the benzoic acid hydrazide (VI) is combined with an excess of the alkyl imidate hydrochloride (VIII) in a suitable organic solvent, such as methanol. The reaction is stirred for about 4 to 20 hours. The condensation product (IX) is then isolated and purified utilizing techniques well known in the art. For example, the reaction is concentrated under vacuum and the residue is treated with a suitable organic solvent, such as diethyl ether. The mixture is then filtered and the filtrate concentrated under vacuum. The residue is again treated with diethyl ether, filtered and concentrated under vacuum to provide the purified condensation product (IX).

In Reaction Scheme B, step B, the condensation product (IX) is subjected to a cyclization reaction with an alkylamine hydrohalide of structure (X) to provide the triazole of formula (.Ib). For example, the condensation product (IX) is dissolved in a suitable organic solvent, such as methanol. It is then treated with an excess of an alkylamine hydrochloride (X) and a suitable base, such as potassium carbonate, in a ratio of alkylamine to base of about 1:1. The reaction is heated at reflux for about 1 to 3 hours. After cooling, the reaction is concentrated under vacuum and the residue is purified by techniques well known in the art. For example, water is added to the residue and the aqueous mixture is extracted with a suitable organic solvent, such as dichloromethane. The combined organic extracts are dried over anhydrous magnesium sulfate, filtered and concentrated under vacuum. The residue is purified by chromatography on silica gel with a suitable eluent, such as methanol/ethyl acetate. The resulting purified material can be further purified by recrystallization from a suitable organic solvent mixture, such as ethyl acetate/hexane to provide the triazole of formula (Ib).

Alternatively, the triazoles of formula (Ib) can be prepared as described in Reaction Scheme C. All substituents, unless otherwise indicated, are previously defined. The reagents and starting materials are readily available to one of ordinary skill in the art.

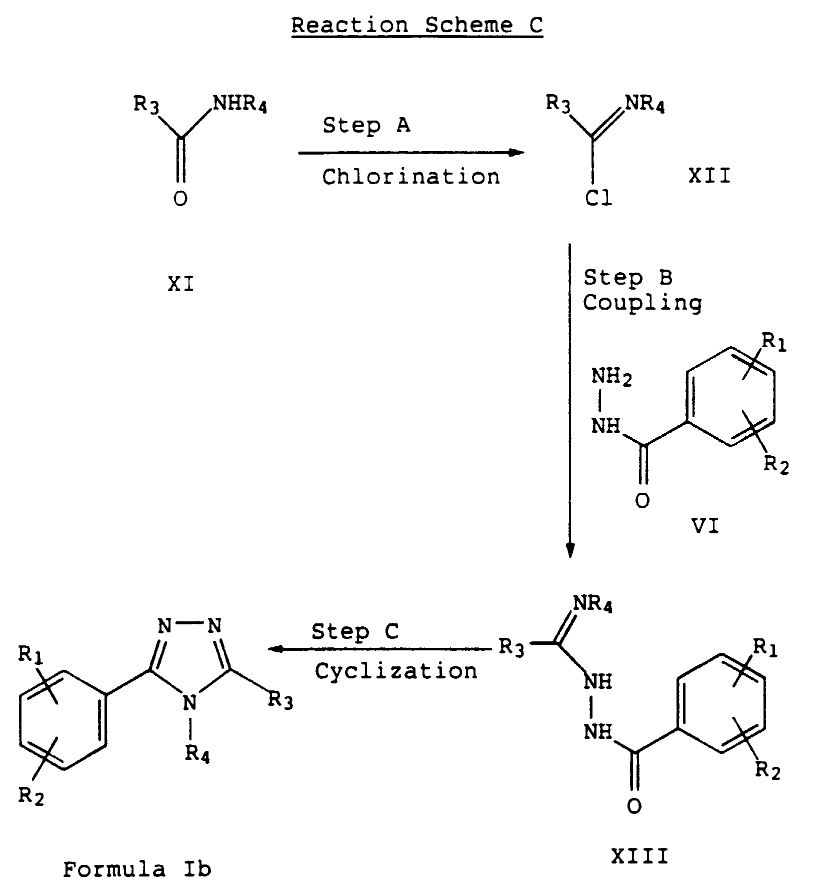

In Reaction Scheme C, step A, an amide of structure (XI) is chlorinated to provide the imidoyl chloride described by structure (XII). For example, the amide (XI) is dissolved in a suitable organic solvent mixture, such as pyridine/choroform. The solution is cooled to a temperature of from 0° to 5°C. A solution of one equivalent of a suitable chlorinating agent, such as phosphorous oxychloride in a suitable organic solvent, such as chloroform is added maintaining the temperature of the reaction below 5°C. The reaction is allowed to stir for about 2 to 4 hours to provide the imidoyl chloride (XII).

In Reaction Scheme C, step B, the imidoyl chloride (XII) is coupled to the benzoic acid hydrazide of structure (VI) to provide the coupled product described by structure (XIII). For example, approximately 0.8 equivalents of the benzoic acid hydrazide (VI) is suspended in a suitable organic solvent, such as chloroform. The above prepared solution of imidoyl chloride (XII) is added dropwise to the suspension over a period of about 30 minutes to 1 hour. The reaction is then allowed to stir for about 4 to 6 hours. The reaction is then diluted with water and the aqueous layer is made basic with a suitable base, such as potassium hydroxide. The basic solution is then extracted with a suitable organic solvent, such as dichloromethane. The combined organic solvents are dried over anhydrous magnesium sulfate, filtered and concentrated under vacuum. The residue is purified by techniques well known in the art. For example the residue is purified by flash chromatography on silica gel with a suitable eluent, such as methanol/dichloromethane to provide the coupled product (XIII).

In Reaction Scheme C, step C, the coupled product (XIII) is cyclized to provide the triazole of formula (Ib). For example, the coupled product (XIII) is dissolved in a suitable organic solvent, such as ethyl acetate. The solution is heated at reflux for about 2 to 4 hours. The reaction is then concentrated under vacuum and the residue is purified by techniques well known in the art. For example, the residue is recrystallized from a suitable solvent mixture, such as ethyl acetate/hexane to provide the triazole of formula (Ib).

The following examples present typical syntheses as described by Reaction Schemes A, B and C. These examples are understood to be illustrative only and are not intended to limit the scope of the invention in any way. As used in the following examples, the following terms have the meanings indicated: "eq." refers to equivalents, "g" refers to grams, "mg" refers to milligrams, "mmol" refers to millimoles, "mL" refers to milliliters, "°C" refers to degrees Celsius, "TLC" refers to thin layer chromatography, "R_{f}" refers to retention factor and "δ" refers to parts per million down field from tetramethylsilane.

### Preparation of 1-(Aroyl)-R₄-Substituted Thiosemicarbazides

### EXAMPLE 1

### 1-(3-Fluorobenzoyl)-4-methylthiosemicarbazide

Dissolve 4-methylthiosemicarbazide (8.48 g, 80.6 mmol) in pyridine (100 mL) at room temperature. Add 3-fluorobenzoyl chloride (9.8 mL, 80 mmol) dropwise to the solution. Stir the reaction overnight at room temperature. Concentrate the reaction under vacuum and wash the residue with water. Collect the solid by filtration, rinse the solid with water and dry the solid by suction.
Recrystallize the solid from ethanol to provide the title compound (8.43 g, 46%) as a colorless powder; mp 199-201°C (dec).

### EXAMPLE 2

### 1-(2-Fluorobenzoyl)-4-methylthiosemicarbazide

Dissolve methyl isothiocyanate (17.2 g, 23.5 mmol) in anhydrous tetrahydrofuran (50 mL) at room temperature. Add to the reaction in one portion a solution of 2-fluorobenzoic acid hydrazide (3.80 g, 24.6 mmol) dissolved in anhydrous tetrahydrofuran (70 mL). Heat the reaction at reflux for 1.5 hours and then place in a freezer. Allow the reaction to stand in the freezer overnight and then collect the solid by filtration. Recrystallize the solid from ethanol/water (9:1) to provide the title compound (4.21 g, 79%) as colorless needles; mp 216-217°C (dec).

### Preparation of 5-aryl-4-substituted-3H-1,2,4-triazole-3-thiones

### EXAMPLE 3

### 5-(3-Fluorophenyl)-4-methyl-3H-1,2,4-triazole-3-thione

Combine 1-(3-fluorobenzoyl)-4-methylthiosemicarbazide (12.0 g, 52.8 mmol) and 1M aqueous sodium bicarbonate (530 mL, 0.53 mol) and heat the mixture at reflux overnight. Then filter the reaction while it is still hot. Allow the filtrate to cool to room temperature and then carefully acidify the filtrate by dropwise addition of concentrated hydrochloric acid (45 mL, 0.54 mol). Cool the mixture in an ice bath and then collect the precipitate by filtration. Wash the solid with water and dry by suction. Recrystallize the solid from isopropanol to provide the title compound (5.64 g, 51%) as colorless, matted needles; mp 150-152°C.

In a similar manner, by substituting a variety of optionally substituted aroyl chlorides and 4-substituted thiosemicarbazides for the reactants of Example 1 or a variety of substituted isothiocyanates and optionally substituted aroylbenzoic acid hydrazides for the reactants of Example 2 and reacting the products according to the general procedures of Example 3, the following intermediate triazole-3-thiones are readily prepared.

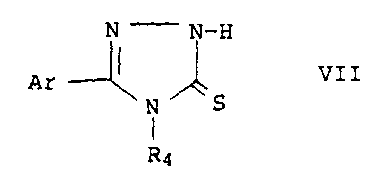

| **Ar** | **R**_{**4**} | **M.P. °C** |
|---|---|---|
| C₆H₅ | CH₃ | 164-166° |
| C₆H₅ | C₆H₅CH₂ | 184-186° |
| 2-ClC₆H₄ | CH₃ | 142-144° |
| 4-ClC₆H₄ | CH₃ | 210-212° |
| 4-ClC₆H₄ | C₂H₅ | 204-206° |
| 2-FC₆H₄ | CH₃ | 137-139° |
| 2-FC₆H₄ | C₂H₅ | 138-140° |
| 3-FC₆H₄ | CH₃ | 150-152° |
| 3-FC₆H₄ | C₂H₅ | 151-153° |
| 3-FC₆H₄ | C₆H₅CH₂ | 183-185° |
| 4-FC₆H₄ | CH₃ | 207-209° |
| 4-CH₃C₆H₄ | CH₃ | 201-203° |
| 4-CH₃OC₆H₄ | CH₃ | 172-174° |
| 4-CH₃OC₆H₄ | C₂H₅ | 173-174° |
| 4-CH₃OC₆H₄ | C₆H₅CH₂ | 201-205° |
| 3-NO₃C₆H₄ | CH₃ | 219-221° |
| 3-NO₃C₆H₄ | C₆H₅CH₂ | 190-192° |
| C₁₀H₇ | CH₃ | 223-225° |

### Preparation of 3-aryl-4-substituted-4H-1,2,4-triazoles

### EXAMPLE 4

### 3-(3-Fluorophenyl)-4-methyl-4H-1,2,4-triazole

Suspend 5-(3-fluorophenyl)-4-methyl-3H-1,2,4-triazole-3-thione (6.00 g, 28.7 mmol) in a 17% solution of nitric acid (63 mL of concentrated nitric acid diluted with 200 mL water). Heat the stirred reaction at reflux for 30 minutes and then allow the reaction to cool to room temperature. Then carefully basify the reaction with aqueous potassium hydroxide to about pH 14. Extract the alkaline solution with dichloromethane (3 × 50 mL). Combine the organic extracts, dry over anhydrous magnesium sulfate, filter and concentrate under vacuum. Recrystallize the residue from acetone/hexane to provide the title compound (4.00 g, 79%); mp 117-119°C.

In a similar manner, by substituting a variety of optionally substituted triazolethiones for the reactants of Example 4 and by substantially following the techniques therein, the following compounds are readily prepared.

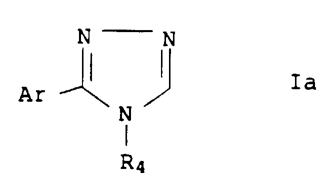

| **Ar** | **R**_{**4**} | **M.P. °C** |
|---|---|---|
| C₆H₅ | CH₃ | 113-116° |
| C₆H₅ | CH₂C₆H₅ | 142-144° |
| 2-ClC₆H₄ | CH₃ | 105-108° |
| 4-ClC₆H₄ | CH₃ | 112-113° |
| 4-ClC₆H₄ | C₂H₅ | 109-111° |
| 2-FC₆H₄ | CH₃ | 88-90° |
| 3-FC₆H₄ | CH₃ | 116-118° |
| 3-FC₆H₄ | C₂H₅ | 86-88° |
| 3-FC₆H₄ | CH₂C₆H₅ | 106-108° |
| 4-FC₆H₄ | CH₃ | 145-146° |
| 2-Br-5-FC₆H₃ | CH₃ | 103-105 |
| 4-CH₃C₆H₄ | CH₃ | 115-117° |
| 4-CH₃OC₆H₄ | CH₃ | 116-118° |
| 4-CH₃OC₆H₄ | C₂H₅ | 97-101° |
| 4-CH₃OC₆H₄ | CH₂C₆H₅ | 105-106° |
| 3-NO₂C₆H₄ | CH₃ | 156-158° |
| 3-NO₂C₆H₄ | CH₂C₆H₅ | 101-102° |
| 2-C₁₀H₇ | CH₃ | 195-197° |

### Preparation of 3-arvl-4,5-disubstituted-4H-1,2,4-triazoles

### EXAMPLE 5

### 4,5-Dimethyl-3-(3-fluorophenyl)-4H-1,2,4-triazole

Combine 3-fluorobenzoic acid hydrazide (4.04 g, 26.2 mmol) and ethyl acetimidate hydrochloride (3.58 g, 29.0 mmol) in methanol (125 mL) with stirring. After 20 hours remove most of the methanol by concentration under vacuum. Add diethyl ether (400 mL) to the concentrate and remove the precipiated ammonium chloride by filtration. Concentrate the filtrate under vacuum and again treat the concentrate with diethyl ether (400 mL). Remove any remaining ammonium chloride by filtration and concentrate the filtrate under vacuum. Dissolve the residue in methanol (170 mL). Add methylamine hydrochloride (5.00 g, 74.0 mmol) and potassium carbonate (10.0 g, 72.3 mmol) to the solution. Heat the reaction at reflux for 1 hour. Then concentrate the reaction under vacuum. Add water to the residue and extract the aqueous mixture with dichloromethane (3 × 150 mL). Combine the organic extracts, dry over anhydrous magnesium sulfate, filter and concentrate under vacuum. Purify the residue by chromatography (5% to 14% methanol/ethyl acetate gradient, silica gel) followed by recrystallization from ethyl acetate/hexane to provide the title compound (2.19 g, 44%) as light yellow needles; mp. 119-121°C.

### EXAMPLE 6

### 4,5-Dimethyl-3-phenyl-4H-1,2,4-triazole

When, in the procedure of Example 5, benzoic acid hydrazide is substituted for 3-fluorobenzoic acid hydrazide, the title compound is obtained. mp=135-137°

### EXAMPLE 7

### 5-Ethyl-3-(3-fluorophenyl)-4-methyl-4H-1,2,4-triazole

Dissolve N-methylpropionamide (1.70 g, 19.5 mmol) in a mixture of pyridine (8 mL) and chloroform (8 mL). Add with stirring a solution of phosphorous oxychloride (3.05 g, 19.9 mmol, in 2 mL of chloroform) maintaining the reaction temperature below 5°C. Stir the reaction for 2 hours, then transfer to a dropping funnel and add this over 30 minutes to a suspension of 3-fluorobenzoic acid hydrazide (2.41 g, 15.6 mmol, in 20 mL of chloroform). Stir the reaction for 4 hours and then pour into water (300 mL). Basify the aqueous mixture with potassium hydroxide and extract with dichloromethane (3 × 200 mL). Combine the organic extracts, dry over anhydrous magnesium sulfate, filter and concentrate under vacuum. Purify the residue by flash chromatography (6.5% methanol/dichloromethane, silica gel). Dissolve the isolated solid in ethyl acetate (75 mL), heat the solution at reflux for approximately 2 hours and then concentrate under vacuum. Recrystallize the residue from ethyl acetate/hexane to provide the title compound (1.00 g, 31%) as colorless plates; mp 124-125°C.

In a similar manner, by substituting a variety of optionally substituted benzoic or naphthoic acid hydrazides and a variety of alkylamines for the reactants of Example 5 or a variety of optionally substituted benzoic or naphthoic acid hydrazides and N-alkyl- or benzyl alkanamides for the reactants of Example 7 and by substantially following the techniques therein, the corresponding 3-aryl-4,5-disubstituted-4H-1,2,4-triazoles are obtained.

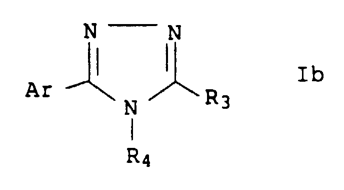

| **Ar** | **R**_{**3**} | **R**_{**4**} | **M.P. °C** |
|---|---|---|---|
| C₆H₅ | CH₃ | CH₃ | 135-137° |
| 3-FC₆H₄ | CH₃ | CH₃ | 119-121° |
| 3-FC₆H₄ | C₂H₅ | CH₃ | 124-125° |

## Claims

1. Use of a compound of the formula 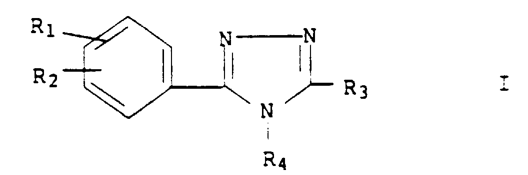 wherein
R₁ and R₂ independently represent hydrogen, halogen, trifluoromethyl, nitro, C₁₋₄ lower alkyl or C₁₋₄ lower alkoxy,
or, together, R1 and R2 represent -CH=CH-CH=CH-, forming a 1- or 2-naphthylenyl ring system;
R₃ represents hydrogen or C₁₋₄ lower alkyl; and
R₄ represents C₁₋₄ lower alkyl, benzyl, or benzyl substituted by one or two groups selected from halogen, trifluoromethyl, nitro, C₁₋₄ lower alkyl or
C₁₋₄ lower alkoxy for the preparation of a pharmaceutical composition for the enhancement of memory and cognition or for treating Alzheimer's disease or the Wernicke-Korsakoff syndrome.

2. The use of claim 1 wherein R₁ is halogen.

3. The use of claim 2 wherein R₁ is fluoro.

4. The use of claim 1 wherein R₂ is hydrogen.

5. The use of claim 1 wherein R₄ is methyl.

6. The use of claim 1 wherein R₄ is benzyl.

7. The use of claim 1 wherein R₃ is hydrogen.

8. The use of claim 1 or 5 wherein R₃ is methyl.

9. The use of claim 5, said compound being 3-(3-fluorophenyl)-4-methyl-4H-1,2,4-triazole.

10. A compound of the formula 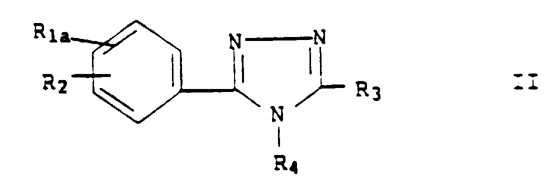 wherein
R₁ₐ represents halogen, trifluoromethyl, nitro, C₁₋₄ lower alkyl or C₁₋₄ lower alkoxy; and R₂ represents hydrogen, halogen, trifluoromethyl, nitro, C₁₋₄ lower alkyl or C₁₋₄ lower alkoxy,
or, together, R₁ₐ and R₂ represent -CH=CH-CH=CH-, forming a 1- or 2-naphthylenyl ring system;
R₃ represents hydrogen or C₁₋₄ lower alkyl; and
R₄ represents C₁₋₄, lower alkyl, benzyl, or benzyl substituted by one or two groups selected from halogen, trifluoromethyl, nitro, C₁₋₄ lower alkyl or C₁₋₄ lower alkoxy,
with the proviso that when R₁ₐ represents 4-chloro and R₂ and R₃ both represent hydrogen, R₄ is other than ethyl.

11. A compound of claim 10 wherein R₁ₐ is halogen.

12. A compound of claim 11 wherein R₁ₐ is fluoro.

13. A compound of claim 10 wherein R₂ is hydrogen.

14. A compound of claim 10 wherein R₄ is methyl.

15. A compound of claim 10 wherein R₄ is benzyl.

16. A compound of claim 10 wherein R₃ is hydrogen.

17. A compound of claim 10 or 14 wherein R₃ is methyl.

18. A compound of claim 14, said compound being 3-(3-fluorophenyl)-4-methyl-4H-1,2,4-triazole.

19. A pharmaceutical composition comprising a therapeutically effective amount of a compound of the formula 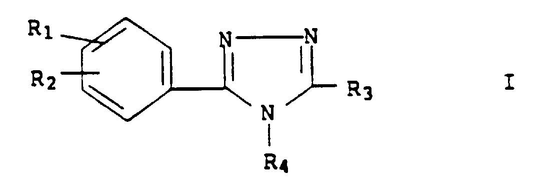 wherein
R₁ and R₂ independently represent hydrogen, halogen, trifluoromethyl, nitro, C₁₋₄ lower alkyl or C₁₋₄ lower alkoxy,
or, together, R₁ and R₂ represent -CH=CH-CH=CH-, forming a 1- or 2-naphthylenyl ring system;
R₃ represents hydrogen or C₁₋₄ lower alkyl; and
R₄ represents C₁₋₄ lower alkyl, benzyl, or benzyl substituted by one or two groups selected from halogen, trifluoromethyl, nitro, C₁₋₄ lower alkyl or C₁₋₄ lower alkoxy,
with the proviso that when R₁ represents 4-chloro and R₂ and R₃ both represent hydrogen, R₄ is other than ethyl, in admixture or otherwise in association with one or more pharmaceutically acceptable carriers or excipients.

20. The use in the manufacture of a medicament of a compound as defined in any one of claims 10 to 18 for enhancement of memory and cognition or for treating a patient afflicted with a Alzheimer's disease or Wernicke-Korsakoff syndrome.

## Patentansprüche

1. Verwendung einer Verbindung der Formel 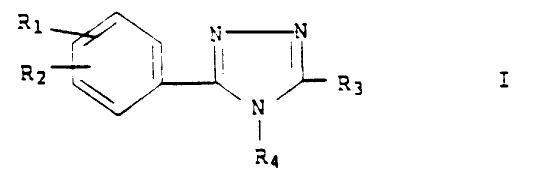 in der
R₁ und R₂ unabhängig ein Wasserstoff-, Halogenatom, eine Trifluormethyl-, Nitrogruppe, einen C₁₋₄-Niederalkyl- oder C₁₋₄-Niederalkoxyrest darstellen, oder R₁ und R₂ zusammen -CH=CH-CH=CH- darstellen, wobei sie ein 1- oder 2-Naphthylenylringsystem bilden;
R₃ ein Wasserstoffatom oder einen C₁₋₄-Niederalkylrest darstellt; und
R₄ einen C₁₋₄-Niederalkylrest, eine Benzylgruppe oder eine Benzylgruppe darstellt, die mit einem oder zwei Resten, ausgewählt aus Halogenatomen, Trifluormethyl-, Nitrogruppen, C₁₋₄-Niederalkyl- oder C₁₋₄-Niederalkoxyresten, substituiert ist, zur Herstellung eines Arzneimittels zur Verbesserung des Gedächtnisses und der Kognition und zur Behandlung von Alzheimer-Krankheit oder des Wernicke-Korsakoff-Syndroms.

2. Verwendung nach Anspruch 1, wobei R₁ ein Halogenatom ist.

3. Verwendung nach Anspruch 2, wobei R₁ ein Fluoratom ist.

4. Verwendung nach Anspruch 1, wobei R₂ ein Wasserstoffatom ist.

5. Verwendung nach Anspruch 1, wobei R₄ eine Methylgruppe ist.

6. Verwendung nach Anspruch 1, wobei R₄ eine Benzylgruppe ist.

7. Verwendung nach Anspruch 1, wobei R₃ ein Wasserstoffatom ist.

8. Verwendung nach Anspruch 1 oder 5, wobei R₃ eine Methylgruppe ist.

9. Verwendung nach Anspruch 5, wobei die Verbindung 3-(3-Fluorphenyl)-4-methyl-4H-1,2,4-triazol ist.

10. Verbindung der Formel 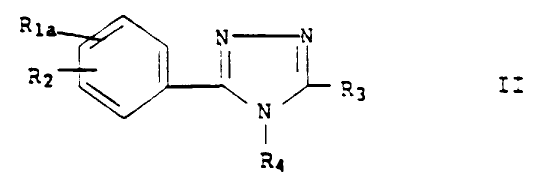 in der
R₁ₐ ein Halogenatom, eine Trifluormethyl-, Nitrogruppe, einen C₁₋₄-Niederalkyl- oder C₁₋₄-Niederalkoxyrest darstellt; und R₂ ein Wasserstoff-, Halogenatom, eine Trifluormethyl-, Nitrogruppe, einen C₁₋₄-Niederalkyl- oder C₁₋₄-Niederalkoxyrest darstellt,
oder R₁ₐ und R₂ zusammen -CH=CH-CH=CH- darstellen, wobei sie ein 1- oder 2-Naphthylenylringsystem bilden;
R₃ ein Wasserstoffatom oder einen C₁₋₄-Niederalkylrest darstellt; und
R₄ einen C₁₋₄-Niederalkylrest, eine Benzylgruppe oder eine Benzylgruppe darstellt, die mit einem oder zwei Resten, ausgewählt aus Halogenatomen, Trifluormethyl-, Nitrogruppen, C₁₋₄-Niederalkyl- oder C₁₋₄-Niederalkoxyresten, substituiert ist,
mit der Maßgabe, daß, wenn R₁ₐ ein 4-Chloratom darstellt und R₂ und R₃ beide ein Wasserstoffatom darstellen, R₄ keine Ethylgruppe ist.

11. Verbindung nach Anspruch 10, in der R₁ₐ ein Halogenatom ist.

12. Verbindung nach Anspruch 11, in der R₁ₐ ein Fluoratom ist.

13. Verbindung nach Anspruch 10, in der R₂ ein Wasserstoffatom ist.

14. Verbindung nach Anspruch 10, in der R₄ eine Methylgruppe ist.

15. Verbindung nach Anspruch 10, in der R₄ eine Benzylgruppe ist.

16. Verbindung nach Anspruch 10, in der R₃ ein Wasserstoffatom ist.

17. Verbindung nach Anspruch 10 oder 14, in der R₃ eine Methylgruppe ist.

18. Verbindung nach Anspruch 14, wobei die Verbindung 3-(3-Fluorphenyl)-4-methyl-4H-1,2,4-triazol ist.

19. Arzneimittel, umfassend eine therapeutisch wirksame Menge einer Verbindung der Formel 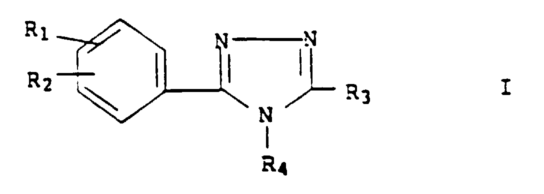 in der
R₁ und R₂ unabhängig ein Wasserstoff-, Halogenatom, eine Trifluormethyl-, Nitrogruppe, einen C₁₋₄-Niederalkyl- oder C₁₋₄-Niederalkoxyrest darstellen, oder R₁ und R₂ zusammen -CH=CH-CH=CH- darstellen, wobei sie ein 1- oder 2-Naphthylenylringsystem bilden;
R₃ ein Wasserstoffatom oder einen C₁₋₄-Niederalkylrest darstellt; und
R₄ einen C₁₋₄-Niederalkylrest, eine Benzylgruppe oder eine Benzylgruppe darstellt, die mit einem oder zwei Resten, ausgewählt aus Halogenatomen, Trifluormethyl-, Nitrogruppen, C₁₋₄-Niederalkyl- oder C₁₋₄-Niederalkoxyresten, substituiert ist,
mit der Maßgabe, daß, wenn R₁ ein 4-Chloratom darstellt und R₂ und R₃ beide ein Wasserstoffatom darstellen, R₄ keine Ethylgruppe ist,
in einem Gemisch oder einer anderen Verbindung mit einem oder mehreren pharmazeutisch verträglichen Trägern oder Exzipienten.

20. Verwendung einer Verbindung nach einem der Ansprüche 1 bis 18 bei der Herstellung eines Medikaments zur Verbesserung des Gedächtnisses und der Kognition oder zur Behandlung eines von Alzheimer-Krankheit oder WernickeKorsakoff-Syndrom betroffenen Patienten.

## Revendications

1. Utilisation d'un composé de formule: 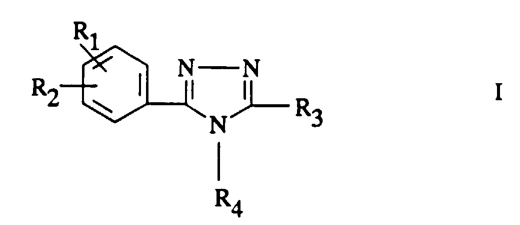 dans laquelle :
R₁ et R₂ représentent indépendamment hydrogène, halogène, trifluorométhyle, nitro, C₁-C₄ alkyle inférieur ou C₁-C₄ alcoxy inférieur,
ou, ensemble, R₁ et R₂ représentent -CH=CH-CH=CH-, formant un système cyclique 1- ou 2-naphtalényle ;
R₃ représente hydrogène ou C₁-C₄ alkyle inférieur ; et
R₄ représente C₁-C₄ alkyle inférieur, benzyle ou benzyle substitué par un ou deux groupes choisis parmi halogène, trifluorométhyle, nitro, C₁-C₄ alkyle inférieur ou C₁-C₄ alcoxy inférieur pour la préparation d'une composition pharmaceutique destinée à l'amélioration de la mémoire et de la cognition ou au traitement de la maladie d'Alzheimer ou du syndrome de Wemicke-Korsakoff.

2. Utilisation selon la revendication 1 dans laquelle R₁ est halogène.

3. Utilisation selon la revendication 2 dans laquelle R₁ est fluoro.

4. Utilisation selon la revendication 1 dans laquelle R₂ est hydrogène.

5. Utilisation selon la revendication 1 dans laquelle R₄ est méthyle.

6. Utilisation selon la revendication 1 dans laquelle R₄ est benzyle.

7. Utilisation selon la revendication 1 dans laquelle R₃ est hydrogène.

8. Utilisation selon la revendication 1 ou 5 dans laquelle R₃ est méthyle.

9. Utilisation selon la revendication 5, dans laquelle ledit composé est le 3-(3-fluorophényl)-4-méthyl-4H-1,2,4-triazole.

10. Composé de formule: 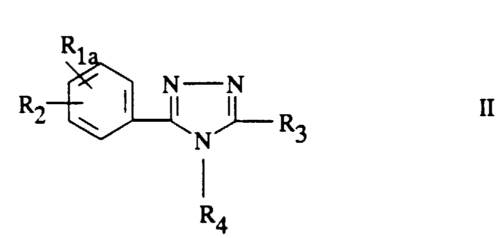 dans laquelle
R₁ₐ représente halogène, trifluorométhyle, nitro, C₁-C₄ alkyle inférieur ou C₁-C₄ alcoxy inférieur; et R₂ représente hydrogène, halogène, trifluorométhyle, nitro, C₁-C₄ alkyle inférieur ou C₁-C₄ alcoxy inférieur,
ou, ensemble, R₁ₐ et R₂ représente -CH=CH-CH=CH-, formant un système cyclique 1- ou 2-naphtalényle ;
R₃ représente hydrogène ou C₁-C₄ alkyle inférieur ; et
R₄ représente C₁-C₄ alkyle inférieur, benzyle ou benzyle substitué par un ou deux groupes choisis parmi halogène, trifluorométhyle, nitro, C₁-C₄ alkyle inférieur ou C₁-C₄ alcoxy inférieur,
à condition que lorsque R₁ₐ représente 4-chloro et R₂ et R₃ représentent tous les deux hydrogène, R₄ soit autre qu'éthyle.

11. Composé selon la revendication 10 dans lequel R₁ₐ est halogène.

12. Composé selon la revendication 11 dans lequel R₁ₐ est fluoro.

13. Composé selon la revendication 10 dans lequel R₂ est hydrogène.

14. Composé selon la revendication 10 dans lequel R₄ est méthyle

15. Composé selon la revendication 10 dans lequel R₄ est benzyle.

16. Composé selon la revendication 10 dans lequel R₃ est hydrogène.

17. Composé selon la revendication 10 ou 14 dans lequel R₃ est méthyle.

18. Composé selon la revendication 14, ledit composé étant le 3-(3-fluorophényl)-4-méthyl-4H-1,2,4-triazole.

19. Composition pharmaceutique comprenant une quantité thérapeutiquement efficace d'un composé de formule: 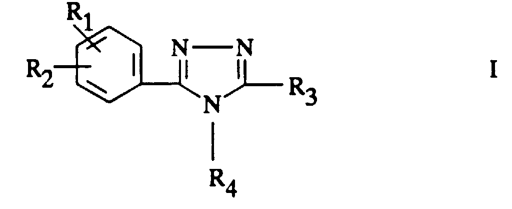 dans laquelle
R₁ et R₂ représentent indépendamment hydrogène, halogène, trifluorométhyle, nitro, C₁-C₄ alkyle inférieur ou C₁-C₄ alcoxy inférieur,
ou, ensemble, R₁ et R₂ représentent -CH=CH-CH=CH-, formant un système cyclique 1- ou 2-naphtalényle ;
R₃ représente hydrogène ou C₁-C₄ alkyle inférieur; et
R₄ représente C₁-C₄ alkyle inférieur, benzyle ou benzyle substitué par un ou deux groupes choisis parmi halogène, trifluorométhyle, nitro, C₁-C₄ alkyle inférieur ou C₁-C₄ alcoxy inférieur,
à condition que lorsque R₁ représente 4-chloro et R₂ et R₃ représentent tous les deux hydrogène, R₄ soit autre qu'éthyle, en mélange ou autrement en association avec un ou plusieurs supports ou excipients pharmaceutiquement acceptables.

20. Utilisation d'un composé tel que défini dans l'une quelconque des revendications 10 à 18 pour la fabrication d'un médicament destiné à l'amélioration de la mémoire et de la cognition ou au traitement d'un patient affligé d'une maladie d'Alzheimer ou du syndrome de Wernicke-Korsakoff.
